# EUROPÄISCHE PATENTSCHRIFT

(11) **EP 2 306 893 B1**
(45) Veröffentlichungstag und Bekanntmachung des Hinweises auf die Patenterteilung: **04.01.2012**
(21) Anmeldenummer: 09734728.0
(22) Anmeldetag: 21.04.2009
(51) Int. Cl.: A61B 5/00, G01N 33/487

(54) **TESTSYSTEM**
TESTING SYSTEM
SYSTÈME DE TEST

(30) Priorität: 23.04.2008 EP 08155024
(43) Veröffentlichungstag der Anmeldung: 13.04.2011
(73) Patentinhaber: F. Hoffmann-La Roche AG, 4070 Basel (CH); Roche Diagnostics GmbH, 68305 Mannheim (DE)
(72) Erfinder: THOES, Bruno, 66287 Quierschied (DE); MILTNER, Karl, 67227 Frankenthal (DE); HESS, Peter, 67551 Worms (DE); IHLE, Günther, 69256 Mauer (DE); KOCH, Martin, 68519 Viernheim (DE); SCHERER, Jörg, 73432 Aalen (DE); SACHERER, Klaus-Dieter, 67281 Kirchheim (DE); PIEGSA, Ralf, 70565 Stuttgart (DE)
(74) Vertreter: Pfiz, Thomas
(86) Internationale Anmeldenummer: PCT/EP2009/054702
(87) Internationale Veröffentlichungsnummer: WO 2009/130195

(56) Entgegenhaltungen:
- EP-A- 1 424 040
- WO-A-2008/022999

## Beschreibung

Die Erfindung betrifft ein Testsystem, insbesondere zur Durchführung von Blutzuckertests, mit einer diagnostischen Bandkassette, die ein aufwickelbares analytisches Testband zum Nachweis eines Analyten und ein das Testband aufnehmendes Kassettengehäuse umfasst, und einem Testgerät, das ein Gerätegehäuse zum Einsetzen und Entnehmen der Bandkassette und eine vorzugsweise optische Messeinheit zur Erfassung des Analyten auf dem Testband aufweist.

Für die Selbstdiagnose von Diabetikern werden bislang in der Praxis einzelne Teststreifen eingesetzt, die nach der Applikation einer geringen Probenmenge photometrisch untersucht werden, um den Glucosegehalt in einer Blutprobe möglichst genau und zuverlässig zu bestimmen. Dabei werden die Messstreifen üblicherweise während der Messung mittels Haltestrukturen festgehalten, die Teile des Gehäuses eines Handgeräts sind. Auf diese Weise soll sichergestellt werden, dass das Messfeld auf dem Teststreifen relativ genau zu der abtastenden Messoptik steht, da diese ebenfalls an den Gehäusebauteilen aufgehängt ist. Nach erfolgter Messung werden die benutzten Einzelstreifen aus dem Gerät entnommen und entsorgt. Durch den Verzicht auf eine geräteinterne Entsorgung wird Platz in einem kleinen Handgerät eingespart, und das mit relativ großen Bautoleranzen behaftete Gehäuse muss nicht in weitere primäre Systemfunktionen einbezogen werden. Hierbei ist zu berücksichtigen, dass das Gerätegehäuse üblicherweise mehrere Funktionen gleichzeitig zu erfüllen hat: Umhüllung (Schutz des Systems), Designdarstellung, Positionierung der Einzelkomponenten relativ zueinander und mechanische Versteifung. Diese Verlagerung vieler Funktionen in ein Bauteil generiert mehrere sich widersprechende Anforderungen und führt dazu, dass die Umsetzung der primären Funktionalität (Umhüllung und Designdarstellung) erschwert wird, während gleichzeitig die weiteren funktionellen Ansprüche - mechanische Versteifung und präzise Relativpositionierung der Systembaugruppen - nur unzulänglich erreicht werden.

Um weitere Anwendungsvorteile zu erreichen, wurde bereits vorgeschlagen, eine Vielzahl von Tests auf einem Testband in Form einer Bandkassette bereitzustellen und wieder zu entsorgen. Solche Bandkassetten sollen sich als Einwegteil in kompakte Handgeräte einsetzen lassen, um alle erforderlichen Untersuchungsschritte automatisch und schnell ausführen zu können.

Ausgehend hiervon liegt der Erfindung die Aufgabe zugrunde, die im Stand der Technik vorgeschlagenen Testsysteme weiter zu verbessern und bei kompakter Bauform eine hohe Positioniergenauigkeit und besondere Anwenderfreundlichkeit beim Einsatz von Bandkassetten zu erreichen.

Zur Lösung dieser Aufgabe wird die im Patenanspruch 1 bzw. 9 angegebene Merkmalskombination vorgeschlagen. Der Oberbegriff dieser Ansprüche ist aus WO-A-2008/022999 bekannt. Vorteilhafte Ausgestaltungen und Weiterbildungen der Erfindung ergeben sich aus den abhängigen Ansprüchen.

Die Erfindung geht von dem Gedanken aus, den Messvorgang geometrisch direkt zwischen Messeinheit und über ein Koppelbauteil damit verbundenem Disposable zu realisieren. Dementsprechend wird gemäß der Erfindung vorgeschlagen, dass die Bandkassette auf einer in dem Gerätegehäuse montierten Plattform durch Positionierelemente lösbar fixierbar ist, und dass die Messeinheit auf der Plattform starr befestigt oder damit in Eingriff bringbar ist. Auf diese Weise ist die Messeinheit in definierter Relativposition zu der fixierten Bandkassette angeordnet, ohne dass Gehäusebauteile unmittelbar beteiligt sind. Durch die Plattform wird gleichsam ein mechanisches Rückgrat geschaffen, das den Anbau oder das Andocken der Messeinheit erlaubt und eine in engen Grenzen reproduzierbare Positionierung eines Bandmagazins ermöglicht.

Vorteilhafterweise bildet die Plattform den Boden eines Kassettenfachs in dem Gerätegehäuse, so dass der Anwender die Bandkassette leicht einfügen und entnehmen kann.

Eine weitere vorteilhafte Ausgestaltung sieht vor, dass die Plattform durch eine über Verbindungsmittel in dem Gerätegehäuse gehaltene, mechanisch eigenstabile Trägerplatte gebildet ist. Dies lässt sich herstellungstechnisch besonders vorteilhaft dadurch realisieren, dass die Plattform als Outsert-Moulding-Teil aus einem Metallträger und daran angeformten Kunststoffteilen besteht.

Für eine kippfreie ebene Abstützung der Bandkassette ist es vorteilhaft, wenn die Positionierelemente mehrere, vorzugsweise drei auf der Plattform angeordnete, punkt- oder linienförmig erhabene Auflager umfassen. Weitere Bewegungsfreiheitsgrade lassen sich dadurch einschränken, dass auf der Plattform angeordnete Positionierelemente unter Aufhebung eines Veschiebespiels mit Positionierelementen der Bandkassette paarweise in Eingriff bringbar sind. Ein solches Verschiebespiel, dass eine einfache Vorpositionierung ermögliche soll, liegt vorteilhafterweise zwischen 1 mm und 3mm.

In einer bevorzugten Ausführung umfassen die Positionierelemente mindestens einen Positionierdorn, der an seiner Basis zylindrisch ausgebildet ist und sich zu seinem freien Ende hin verjüngt, um somit ein intuitives Aufsetzen der Kassette für den Anwender zu ermöglichen. In diesem Zusammenhang ist es auch vorteilhaft, wenn die Positionierelemente mindestens ein Langloch oder Rundloch für den Eingriff eines Positionierdorns aufweisen.

Ein weiterer bevorzugter Aspekt der Erfindung liegt darin, dass die Bandkassette beim Einsetzen unter Einwirkung einer Federanordnung aus einer losen Einlegestellung in eine definierte gerätefeste Messstellung gelangt. Dadurch wird eine besonders einfache Handhabung durch den Anwender möglich, wobei eine solche wegabhängig geschaltete Arretierung der Kassette auch in dem kleinen Bauraum eines Handgeräts einfach realisierbar ist.

Eine weitere Verbesserung in der Handhabung ergibt sich dadurch, dass die Bandkassette in einer einachsigen Bewegung in Richtung einer Einlegeachse in das Testgerät einsetzbar ist.

Für eine weitgehend automatische Endpositionierung ist es von Vorteil, wenn die Federanordnung mindestens eine in Richtung der Einlegeachse komprimierbare Druckfeder aufweist, und wenn die Federanordnung durch Schließen eines Gehäusedeckels des Testgeräts betätigbar ist. Dies lässt sich dadurch verwirklichen, dass eine an einem Gerätedeckel des Testgeräts innenseitig abstehende Druckfeder der Federanordnung beim Schließen des Gerätedeckels auf der eingesetzten Bandkassette abgestützt ist.

Zur Realisierung weiterer Funktionen ist es vorteilhaft, wenn eine Druckfeder der Federanordnung an einer Aufwickelspule der Bandkassette angreift.

Die gewünschte Endposition lässt sich in engen Toleranzgrenzen dadurch definieren, dass die Federanordnung mindestens eine quer zur Einlegeachse auslenkbare Andrückfeder zur Herstellung einer Klemmverbindung der Bandkassette aufweist. Eine weitere Verbesserung sieht vor, dass die vorzugsweise als Blattfeder ausgebildete Andrückfeder über eine Anlaufschräge der Bandkassette unter Vorspannung setzbar ist.

Um ein Verkanten im Gerät zu vermeiden, ist es vorteilhaft, wenn die eingesetzte Bandkassette an mindestens zwei beabstandeten Stützstellen durch die Federanordnung federbelastet ist.

Eine besonders kompakte Bauform lässt sich dadurch erreichen, dass die Messeinheit in Richtung der Einlegeachse in einen Freiraum der in das Testgerät eingesetzten Bandkassette eingreift. Hierbei ist es auch von Vorteil, wenn die Messeinheit durch einen auf der Plattform montierten, reflektionsphotometrisch arbeitenden Messkopf gebildet ist, wobei der Strahlengang des Messkopfs quer zur Bandtransportrichtung der in der Messstellung befindlichen Bandkassette verläuft.

Vorteilhafterweise ist die Bandkassette über eine Kupplung mit einem geräteseitigen Bandantrieb verbindbar, wobei die Kupplung Formschlusselemente aufweist, die über eine Ausweichbewegung beim Einsetzen der Bandkassette aus einer getrennten Ausgangsstellung in eine drehschlüssige Eingriffstellung bringbar sind. Durch die Ausweichbewegung können Totlagen der Formschlusselemente vermieden bzw. überwunden werden. Somit lässt sich ein Blockieren der Kassette beim Einlegen verhindern. Zugleich wird sichergestellt, dass auch eine zur definierten Testpositionierung unter permanenter Bandspannung gehaltene Bandkassette problemlos in das Gerät einsetzbar ist.

Gegenstand der Erfindung ist auch eine Bandkassette mit einer Abwickelspule und einer Aufwickelspule sowie einer Bandführung zum definierten Transport eines analytischen Testbands zwischen den Spulen, wobei die Bandkassette dazu ausgebildet bzw. geeignet und bestimmt ist, als Verbrauchsmittel in ein erfindungsgemäßes Testsystem eingesetzt zu werden.

Im Folgenden wird die Erfindung anhand eines in der Zeichnung schematisch dargestellten Ausführungsbeispiels näher erläutert. Es zeigen:
- Fig. 1: ein Blutzuckertestgerät mit einer integrierten Plattform zum Aufnehmen einer Bandkassette in perspektivischer Darstellung;
- Fig. 2: die Plattform nach Fig. 1 vergrößerter perspektivischer Ansicht;
- Fig. 3: eine Bandkassette als Testmittel für das Blutzuckertestgerät in teilweise aufgebrochener Ansicht von unten;
- Fig. 4: das Testsystem bestehend aus Blutzuckertestgerät und eingesetzter Bandkassette in schaubildlicher Darstellung.

Das in der Zeichnung dargestellte Blutzuckertestgerät 10 ermöglicht in Form eines Handgeräts den Einsatz einer Bandkassette 12 als analytisches Verbrauchsmittel zur Durchführung einer Vielzahl von Selbsttests mit vor Ort vom Patienten selbst gewonnenen Blutproben.

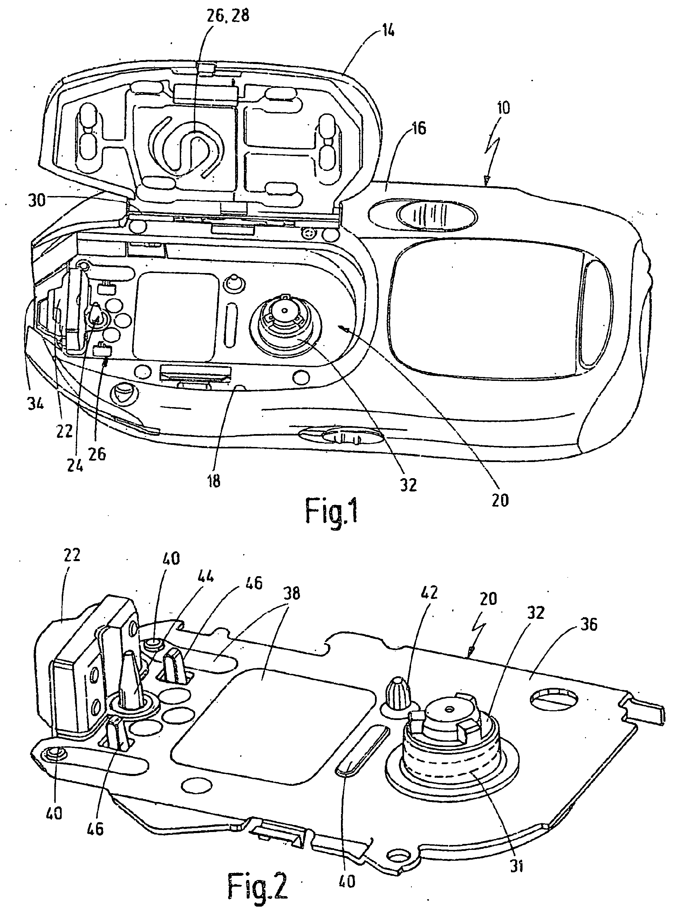

Fig. 1 zeigt das Blutzuckertestgerät 10 bei geöffnetem Deckel 14 des Gehäuses 16 zur Freigabe des Kassettenfachs 18. Das Kassettenfach 18 wird bodenseitig durch eine Plattform 20 begrenzt, die ein Gerätechassis zur definierten Befestigung einer optischen Messeinheit 22 und zur positionsgenauen Lagerung der Bandkassette 12 relativ zu der Messeinheit bildet. Hierfür sind auf der Plattform 20 und an der Bandkassette 12 mehrere Positionierelemente 24 angeordnet, die in Verbindung mit einer Federanordnung 26 neben der exakten Positionierung auch eine einfache Handhabung beim Kassettentausch gewährleisten.

Die Federanordnung 26 umfasst eine Deckelfeder 28, die als innenseitig abstehendes Ringgebilde aus einem Blechmaterial ausgestanzt und vorgebogen ist. Der an einem Scharnier 30 angelenkte Deckel 14 lässt sich gegen eine eingesetzte Bandkassette 12 zuschwenken, so dass die Deckelfeder 28 eine zu der Plattform 20 hin gerichtete Federkraft auf die Bandkassette ausübt. Auf der Plattform 20 ist ein durch Schraubenfeder 31 axial gefederter Drehantriebszapfen 32 eines Bandantriebs mit der Bandkassette in Eingriff bringbar. Durch den Bandantrieb kann Testbandmaterial in den Bereich einer Gehäuseöffnung 34 transportiert werden, um dort nach Beaufschlagung mit Blutflüssigkeit einen Glucosenachweis durchzuführen. Einzelheiten des Testablaufs sind beispielsweise aus der EP-A 1878379 bekannt, worauf hier ausdrücklich Bezug genommen wird.

Fig. 2 zeigt die Plattform 20 mit den daran aufgebauten Funktionsteilen als eigenständige Baueinheit des Testgeräts 10. Die Plattform 20 ist als Outsert-Moulding-Teil in Outsert-Technik aus einem Metallträger 36 und daran angespritzten Kunststoffteilen 38 gebildet. In der Outsert-Technik wird der Metallträger in den Werkzeughohlraum eines schließbaren Formwerkzeugs eingelegt und vorzugsweise durch Spritzgießen mit aushärtbarer Kunststoffmasse verbunden. Dabei wird der ausgeformte Kunststoff über Hinterschneidungen und Durchbrüche an dem Metallträger fest verankert. Das Verfahren als solches ist dem Fachmann bekannt, so dass weitere Verfahrensdetails hier nicht beschrieben werden müssen.

Die Plattform 20 kann als zentrales Element im Testsystem alle Funktionsteile zur Sicherstellung der wesentlichen Gerätefunktionen aufnehmen und somit zu einer Zentralbaugruppe werden. Zweckmäßig können dann an dieser Zentralbaugruppe alle Funktionen im Zuge der Geräteproduktion eigenständig getestet werden. Vorteilhafterweise sind alle für die Messfunktion der Messeinheit 22 notwendigen Bauteile auf der Plattform 20 aufgebaut, so dass die Messfunktion unabhängig von einem Einbau in das Gerätegehäuse 16 prüfbar ist. Ein weiterer Vorteil ergibt sich dahingehend, dass Änderungen beispielsweise am Design des Gerätegehäuses 16 nicht zwingend mit einer geometrischen Veränderung der Plattform 20 und der darauf aufgebauten Funktionsteile einhergehen müssen.

Zur kippfreien Abstützung der Bandkassette 12 sind auf der Plattform 20 drei nach oben abstehende Auflager 40 aus Kunststoff angeformt. Diese spannen geometrisch eindeutig eine Positionierebene auf. Die Arretierung in dieser Ebene erfolgt über zwei Dorne 42, 44, die in Öffnungen der Bandkassette 12 eingreifen. Wie es nachstehend näher erläutert wird, sorgen dabei zwei als U-förmige Blattfedern ausgebildete Andrückfedern 46 der Federanordnung 26 für eine Klemmverbindung in der vorgesehenen Endposition.

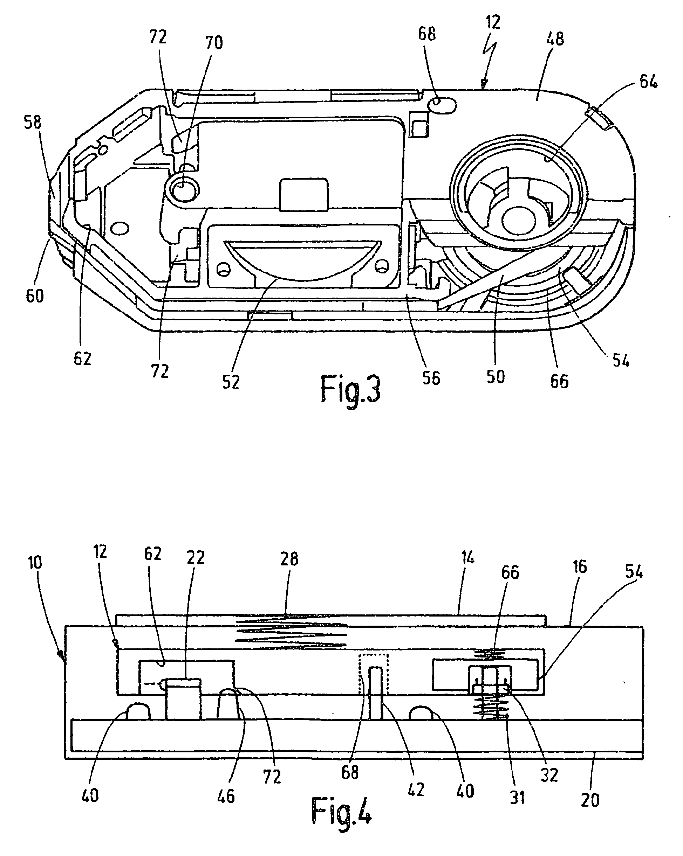

Fig. 3 zeigt die Bandkassette 12 von unten mit aufgebrochener Längshälfte. In dem Kassettengehäuse 48 ist ein Testband 50 von einer Vorratsspule 52 abziehbar und auf eine Aufwickelspule 54 aufwickelbar. Zwischen den Spulen wird das Testband 50 durch einen Bandführungsrahmen 56 über eine Applikationsspitze 58 umgelenkt, um eine vorderseitige Applikation von Körperflüssigkeit (Blut bzw. Gewebeflüssigkeit) und eine rückseitige reflektometrische Vermessung zu erlauben. Hierzu sind auf dem Testband 12 mit Trockenchemikalien versehene Testfelder 60 abschnittsweise aufgebracht, die auf den Analyten (Glucose) in der applizierten Blutflüssigkeit reagieren und bei rückseitiger Beleuchtung zu einer messbaren Veränderung der Lichtrückstrahlung führen. Für die rückseitige Vermessung ist hinter der Applikationsspitze 58 ein Freiraum 62 für den Eingriff des Messkopfs 22 freigehalten.

Zum Vorspulen des Testbands 50 lässt sich die Nabe 64 der Aufwickelspule 54 drehschlüssig mit dem Antriebszapfen 32 verbinden. Eine am Kassettengehäuse 48 deckelseitig abgestützte Druckfeder 66 wirkt dabei entgegen der Antriebszapfenfeder 31.

Als weitere Positionierelemente sind in dem Kassettengehäuse 48 ein Langloch 68 und ein Rundloch 70 zum Aufstecken auf die Dorne 42, 44 der Plattform 20 vorgesehen. Die Dorne gewährleisten mit ihrer verjüngten Spitze beim Aufsetzen der Bandkassette zunächst ein größeres Spiel, während im Bereich der zylindrischen Dornbasis nur noch ein reduzierter Luftspalt frei bleibt. Zur Aufhebung dieses verbleibenden Freiraums sind am Kassettengehäuse zwei Anlaufschrägen 72 für die Andrückfedern 46 angeformt.

Wie anhand Fig. 4 veranschaulicht, ermöglicht die vorstehend beschriebene Anordnung ein vereinfachtes Einsetzen der Bandkassette 12 in das Testgerät 10 und zugleich eine hohe Positioniergenauigkeit in der vorgesehenen Messposition mit einer wegabhängigen Schaltung der Zustände "lose" und "fest" der Kassette im Gerät. Zunächst kann die Bandkassette 12 durch den Anwender in einer senkrecht zu der Plattform 20 verlaufenden linearen Bewegung direkt in das Kassettenfach 18 eingelegt und noch lose auf die verjüngten Dorne 42, 44 aufgesetzt werden. Anschließend wird durch Schließen des Gehäusedeckels 14 unter der Kraft der Deckelfeder 28 die Kassette in ihre gewünschte Endlage gedrückt und dort arretiert. Dabei wirken die verschiedenen Positionierelemente und Federn aufeinander abgestimmt, um die Kassette in engen Toleranzen relativ zur Messeinheit 22 zu positionieren.

Die Auflager 40, 42 definieren die Positionierebene und reduzieren somit drei der sechs möglichen Freiheitsgrade für Bewegungsmöglichkeiten der Bandkassette 12. Durch die Kombination von Dorn 44 und Rundloch 70 werden zwei weitere Freiheitsgrade reduziert. Der verbleibende Rotationsfreiheitsgrad wird durch Dorn 42 in Verbindung mit Langloch 68 unterbunden.

Um ein anfängliches Verklemmen beim Einlegen zu vermeiden, erlauben die Positionierelementpaare 44, 70 und 42, 68 ein Verschiebespiel in Kassettenlängsrichtung von ca. 1 mm. Dieses Verschiebespiel wird durch die Durchmesserdifferenz zwischen Rundloch 70 und Dorn 44 bestimmt, während das Langloch 68 eine etwas größere Lochlänge aufweist. Um eine reproduzierbare Endposition zu gewährleisten, sorgen die gegen die Schrägen 72 anlaufenden Andrückfedern 46 für eine klemmende tangentiale Anlage des Dorns 44 an seiner Basis. Der Antriebszapfen 32 besitzt hierbei genügend zusätzliches Querspiel, wobei die stirnseitigen Mitnehmer unter der Kraft der Federn 31, 66 einen drehfesten Formschluss mit der Nabe 64 herstellen.

In der eingesetzten Endstellung bzw. Messstellung verläuft der Strahlengang des Messkopfs 22 quer zur Bandtransportrichtung der Bandkassette, wobei durch die punktgenaue Positionierung eine reproduzierbare Erfassung des Testfelds gewährleistet ist.

Wie bereits erwähnt, bildet die Aufwickelspule 54 mit dem Antriebszapfen 32 im eingelegten Zustand der Kassette 12 eine formschlüssige Kupplung. Allgemein ist es von Vorteil, wenn die antreibende Seite (Eingang) und die angetriebene Seite (Ausgang) der Kupplung jeweils Elemente aufweisen, die einerseits eine Zentrierung der beiden Seiten zueinander ermöglichen (koaxiale Ausrichtung) und andererseits eine Drehbewegung der Eingangsseite in eine Drehbewegung der Ausgangsseite übertragen. Die drehschlüssige Verbindung kann dabei durch Formschlusselemente sichergestellt werden, die in der Lage sind, mit ihrem jeweiligen Gegenpart einen Formschluss zu generieren. Dabei greift ein Element der Eingangsseite in eine Lücke zwischen zwei Elementen der Ausgangsseite. Beim Einlegen der Kassette kann es jedoch vorkommen, dass die Formschlusselemente nicht "Zahn auf Lücke" stehen, sondern "Zahn auf Zahn". Diese Orientierung der Elemente von Eingangs- und Ausgangsseite der Formschlusskupplung würde zum Stocken oder Blockieren des Einlegevorganges führen, derart, dass die Kassette nicht die gewünschte Endlage im Gerät erreichen würde. Ohne weitere Abhilfe könnte der Anwender das Gerät folglich nicht in Betrieb nehmen. Um dies zu vermeiden, wird ein Ausweichen eines der Formschlusselemente vorgesehen. In der vorliegenden Konstruktion ist das ausweichende Element in Form des Mitnehmers 32 ein Teil des Gerätes 10. Die gewählte Ausweichbewegung ist axial. Das ausweichende Element ist mittels der als Zylinderfeder ausgebildeten Antriebszapfenfeder 31 axial angefedert.

Unabhängig von dem gewählten Ausführungsbeispiel sind konzeptionell andere Ausführungsformen für diese Funktion denkbar. Das ausweichende Element könnte ebenfalls Teil der Kassette sein (z.B. als Teil innerhalb der Nabe 64). Die Ausweichbewegung könnte radial stattfinden oder auch rotatorisch, durch "Zwangsverdrehen" der Formschlusselemente während des Einlegevorganges, so dass sich die Formschlusselemente von der Stellung "Zahn auf Zahn" auf die Stellung "Zahn auf Lücke" verdrehen. Das ausweichende Element kann wie vorliegend ein separates Bauteil (mit dazugehörender Feder) sein, es kann aber auch ein Teil der Eingangs- oder auch Ausgangsseite sein, derart, dass durch Gestaltung und/oder Materialwahl sowohl ein Ausweichen beim Einlegen und auch das Übertragen von Drehmoment im Betrieb stattfinden kann.

Zum Entnehmen einer verbrauchten Bandkassette 12 muss der Benutzer nur den Gehäusedeckel 14 öffnen, um so ein selbsttätiges Anheben der Kassette durch die vorgespannten Federn 31, 66 und 46 auszulösen. Das Federzusammenspiel gewährleistet dabei ein paralleles Anheben der Kassette an zwei beabstandeten Stützstellen, so dass ein Verkanten im zylindrischen Bereich der Positionierdorne 42, 44 vermieden wird. Anschließend ist es möglich, die Kassette 12 bei nach unten weisendem Deckel 14 allein unter Schwerkraft ohne zusätzliche Krafteinleitung aus dem Kassettenfach 18 herausfallen zu lassen.

## Patentansprüche

1. Testsystem, insbesondere zur Durchführung von Blutzuckertests, mit einer diagnostischen Bandkassette (12), die ein aufwickelbares analytisches Testband (50) zum Nachweis eines Analyten und ein das Testband (50) aufnehmendes Kassettengehäuse (48) umfasst, und einem Testgerät (10), das ein Gerätegehäuse (16) zum Einsetzen und Entnehmen der Bandkassette (12) und eine vorzugsweise optische Messeinheit (22) zur Erfassung des Analyten auf dem Testband (50) aufweist, **dadurch gekennzeichnet, dass** die Bandkassette (12) auf einer in dem Gerätegehäuse (16) montierten Plattform (20) durch Positionierelemente (24) lösbar fixierbar ist, und dass die Messeinheit (22) auf der Plattform (20) starr befestigt oder damit in Eingriff bringbar ist.

2. Testsystem nach Anspruch 1, **dadurch gekennzeichnet, dass** die Plattform (20) den Boden eines Kassettenfachs (18) in dem Gerätegehäuse (16) bildet.

3. Testsystem nach Anspruch 1 oder 2, **dadurch gekennzeichnet, dass** die Plattform (20) durch eine über Verbindungsmittel in dem Gerätegehäuse (16) gehaltene, mechanisch eigenstabile Trägerplatte (36) gebildet ist.

4. Testsystem nach einem der Ansprüche 1 bis 3, **dadurch gekennzeichnet, dass** die Plattform (20) als Outsert-Moulding- Teil aus einem Metallträger (36) und daran angeformten Kunststoffteilen (38) besteht.

5. Testsystem nach einem der Ansprüche 1 bis 4, **dadurch gekennzeichnet, dass** die Positionierelemente (24) mehrere, vorzugsweise drei auf der Plattform (20) angeordnete, punkt- oder linienförmig erhabene Auflager (40) für eine kippfreie Abstützung der Bandkassette (12) umfassen.

6. Testsystem nach einem der Ansprüche 1 bis 5, **dadurch gekennzeichnet, dass** auf der Plattform (20) angeordnete Positionierelemente (24;42,44) unter Aufhebung eines Veschiebespiels mit Positionierelementen (24;68,70) der Bandkassette (12) paarweise in Eingriff bringbar sind.

7. Testsystem nach einem der Ansprüche 1 bis 6, **dadurch gekennzeichnet, dass** die Positionierelemente (24) mindestens einen Positionierdorn (42,44) umfassen, der an seiner Basis zylindrisch ausgebildet ist und sich zu seinem freien Ende hin verjüngt.

8. Testsystem nach einem der Ansprüche 1 bis 7, **dadurch gekennzeichnet, dass** die Positionierelemente (24) mindestens ein Langloch (68) oder Rundloch (70) für den Eingriff eines Positionierdorns (42,44) aufweisen.

9. Testsystem, insbesondere zur Durchführung von Blutzuckertests, mit einer diagnostischen Bandkassette (12), die ein aufwickelbares analytisches Testband (50) zum Nachweis eines Analyten und ein das Testband (50) aufnehmendes Kassettengehäuse (48) umfasst, und einem Testgerät (10), das ein Gerätegehäuse (16) zum Einsetzen und Entnehmen der Bandkassette (12) und eine vorzugsweise optische Messeinheit (22) zur Erfassung des Analyten auf dem Testband (50) aufweist, **dadurch gekennzeichnet, dass** die Bandkassette (12) beim Einsetzen unter Einwirkung einer Federanordnung (26) aus einer losen Einlegestellung in eine definierte gerätefeste Messstellung gelangt.

10. Testsystem nach einem der Ansprüche 1 bis 9, **dadurch gekennzeichnet, dass** die Bandkassette (12) in einer einachsigen Bewegung in Richtung einer Einlegeachse in das Testgerät (10) einsetzbar ist.

11. Testsystem nach Anspruch 9 oder 10, **dadurch gekennzeichnet, dass** die Federanordnung (26) durch Schließen eines Gehäusedeckels des Testgeräts (10) betätigbar ist.

12. Testsystem nach Anspruch 11, **dadurch gekennzeichnet, dass** eine an einem Gerätedeckel des Testgeräts (10) innenseitig abstehende Druckfeder (28) der Federanordnung (26) beim Schließen des Gerätedeckels auf der eingesetzten Bandkassette (12) abgestützt ist.

13. Testsystem nach einem der Ansprüche 9 bis 12, **dadurch gekennzeichnet, dass** eine Druckfeder (66) der Federanordnung (26) an einer Aufwickelspule (54) der Bandkassette (12) angreift.

14. Testsystem nach einem der Ansprüche 9 bis 13, **dadurch gekennzeichnet, dass** die Federanordnung (26) mindestens eine quer zur Einlegeachse auslenkbare Andrückfeder (46) zur Herstellung einer Klemmverbindung der Bandkassette (12) aufweist.

15. Testsystem nach einem der Ansprüche 9 bis 14, **dadurch gekennzeichnet, dass** die eingesetzte Bandkassette (12) an mindestens zwei beabstandeten Stützstellen durch die Federanordnung (26) federbelastet ist.

16. Testsystem nach einem der Ansprüche 1 bis 15, **dadurch gekennzeichnet, dass** die Bandkassette (12) über eine Kupplung mit einem geräteseitigen Bandantrieb verbindbar ist, und dass die Kupplung Formschlusselemente (32,64) aufweist, die über eine Ausweichbewegung beim Einsetzen der Bandkassette (12) drehschlüssig miteinander in Eingriff bringbar sind.

17. Bandkassette (12) mit einer Abwickelspule (52) und einer Aufwickelspule (54) sowie einer Bandführung (56) zum definierten Transport eines analytischen Testbands (50) zwischen den Spulen (52,54), ausgebildet als Verbrauchsmittel für ein Testsystem nach einem der vorhergehenden Ansprüche.

## Claims

1. Test system in particular for carrying out blood sugar tests with a diagnostic tape cassette (12) which comprises a windable analytical test tape (50) for detecting an analyte and a cassette housing (48) accommodating the test tape (50), and a test device (10) which has a device housing (16) for inserting and removing the tape cassette (12) and a preferably optical measuring unit (22) for detecting the analyte on the test tape (50), **characterized in that** the tape cassette (12) can be detachably fastened by means of positioning elements (24) on a platform (20) mounted in the device housing (16), and that the measuring unit (22) is fixedly attached on the platform (20) or can be engaged therewith.

2. Test system according to claim 1, **characterized in that** the platform (20) forms the base of a cassette compartment (18) in the device housing (16).

3. Test system according to claim 1 or 2, **characterized in that** the platform (20) is formed by a mechanically self-stable support plate (36) which is held in the device housing (16) by connecting means.

4. Test system according to one of the claims 1 to 3, **characterized in that** the platform (20) as an outsert moulding part consists of a metal support (36) and plastic parts (38) formed thereon.

5. Test system according to one of the claims 1 to 4, **characterized in that** the positioning elements (24) comprise several, preferably three point-shaped or linear raised supports (40) that are disposed on the platform (20) for a tilt-free support for the tape cassette (12).

6. Test system according to one of the claims 1 to 5, **characterized in that** the positioning elements (24; 42, 44) arranged on the platform (20) can be engaged in pairs with positioning elements (24; 68, 70) of the tape cassette (12) while nullifying a displacement play.

7. Test system according to one of the claims 1 to 6, **characterized in that** the positioning elements (24) comprise at least one positioning pin (42, 44) the base of which is cylindrical and tapers towards its free end.

8. Test system according to one of the claims 1 to 7, **characterized in that** the positioning elements (24) have at least one oblong hole (68) or round hole (70) for the engagement of a positioning pin (42, 44).

9. Test system in particular for carrying out blood sugar tests with a diagnostic tape cassette (12) which comprises a windable analytical test tape (50) for detecting an analyte and a cassette housing (48) accommodating the test tape (50), and a test device (10) which has a device housing (16) for inserting and removing the tape cassette (12) and a preferably optical measuring unit (22) for detecting the analyte on the test tape (50), **characterized in that** when the tape cassette (12) is inserted, it moves from a loose insertion position into a defined fixed measuring position in the device under the action of a spring arrangement (26).

10. Test system according to one of the claims 1 to 9, **characterized in that** the tape cassette (12) can be inserted into the test device (10) in an uniaxial motion in the direction of an insertion axis.

11. Test system according to claim 9 or 10, **characterized in that** the spring arrangement (26) can be actuated by closing a housing cover of the test device (10).

12. Test system according to claim 11, **characterized in that** a compression spring (28) of the spring arrangement (26) projecting from the inner side of a housing cover of the test device (10) is supported on the inserted tape cassette (12) when the housing cover is closed.

13. Test system according to one of the claims 9 to 12, **characterized in that** a compression spring (66) of the spring arrangement (26) engages with a take-up spool (54) of the tape cassette (12).

14. Test system according to one of the claims 9 to 13, **characterized in that** the spring arrangement (26) has at least one pressure spring (46) which can be deflected crosswise to the insertion axis in order to make a clamp connection of the tape cassette (12).

15. Test system according to one of the claims 9 to 14, **characterized in that** the inserted tape cassette (12) is spring-loaded by the spring arrangement (26) at at least two spaced apart support points.

16. Test system according to one of the claims 1 to 15, **characterized in that** the tape cassette (12) can be connected to a tape drive of the device by means of a coupling and that the coupling has form-fit elements (32, 64) which upon insertion of the tape cassette (12) can be brought into a rotationally locked engagement position with one another by a yielding movement.

17. Tape cassette (12) with a supply spool (52) and a take-up spool (54) as well as a tape guide (56) for the defined transport of an analytical test tape (50) between the spools (52, 54) which is designed as a disposable for a test system according to one of the previous claims.

## Revendications

1. Système de test, en particulier pour effectuer des tests de glycémie, comprenant une cassette à bande diagnostique (12) qui comporte une bande de test analytique (50) pouvant être embobinée pour la détection d'un analyte et un boîtier de cassette (48) recevant la bande de test (50), et un appareil de test (10) qui comporte un boîtier d'appareil (16) pour la mise en place et le retrait de la cassette à bande (12) et un ensemble de mesure (22), de préférence optique, pour la détection de l'analyte sur la bande de test (50), **caractérisé en ce que** la cassette à bande (12) peut être fixée de manière amovible, par des éléments de positionnement (24), sur une plateforme (20) montée dans le boîtier d'appareil (16), et **en ce que** l'ensemble de mesure (22) est fixé de manière rigide sur la plateforme (20) ou peut être mis en prise avec celle-ci.

2. Système de test selon la revendication 1, **caractérisé en ce que** la plateforme (20) forme le fond d'un compartiment de cassette (18) dans le boîtier d'appareil (16).

3. Système de test selon la revendication 1 ou 2, **caractérisé en ce que** la plateforme (20) est constituée par une plaque support (36) à stabilité mécanique propre, tenue dans le boîtier d'appareil (16) par des moyens de liaison.

4. Système de test selon l'une des revendications 1 à 3, **caractérisé en ce que** la plateforme (20), en tant que pièce moulée in situ, est composée d'un support métallique (36) et de pièces en matière plastique (38) formées sur celui-ci.

5. Système de test selon l'une des revendications 1 à 4, **caractérisé en ce que** les éléments de positionnement (24) comprennent plusieurs, de préférence trois appuis (40) disposés sur la plateforme (20), réalisés en relief sous forme de point ou de ligne, pour un appui sans basculement de la cassette à bande (12).

6. Système de test selon l'une des revendications 1 à 5, **caractérisé en ce que** des éléments de positionnement (24 ; 42, 44) disposés sur la plateforme (20) peuvent, en supprimant un jeu de déplacement, être mis en prise par paire avec des éléments de positionnement (24 ; 68, 70) de la cassette à bande (12).

7. Système de test selon l'une des revendications 1 à 6, **caractérisé en ce que** les éléments de positionnement (24) comprennent au moins un mandrin de positionnement (42, 44), réalisé sous une forme cylindrique à sa base et s'amincissant en direction de son extrémité libre.

8. Système de test selon l'une des revendications 1 à 7, **caractérisé en ce que** les éléments de positionnement (24) présentent au moins un trou oblong (68) ou trou rond (70), pour l'engagement d'un mandrin de positionnement (42, 44).

9. Système de test, en particulier pour effectuer des tests de glycémie, comprenant une cassette à bande diagnostique (12) qui comporte une bande de test analytique (50) pouvant être embobinée pour la détection d'un analyte et un boîtier de cassette (48) recevant la bande de test (50), et un appareil de test (10) qui comporte un boîtier d'appareil (16) pour la mise en place et le retrait de la cassette à bande (12) et un ensemble de mesure (22), de préférence optique, pour la détection de l'analyte sur la bande de test (50), **caractérisé en ce que** la cassette à bande (12) parvient lors de la mise en place, sous l'effet d'un agencement à ressorts (26), d'une position d'insertion libre à une position de mesure définie fixe de l'appareil.

10. Système de test selon l'une des revendications 1 à 9, **caractérisé en ce que** la cassette à bande (12) peut être mise en place dans l'appareil de test (10) en un mouvement uniaxial en direction d'un axe d'insertion.

11. Système de test selon la revendication 9 ou 10, **caractérisé en ce que** l'agencement à ressorts (29) peut être actionné en fermant un couvercle de boîtier de l'appareil de test (10).

12. Système de test selon la revendication 11, **caractérisé en ce qu'**un ressort de pression (28) de l'agencement à ressorts (26), saillant sur le côté intérieur d'un couvercle d'appareil de l'appareil de test (10), est en appui sur la cassette à bande (12) mise en place, lorsque l'on ferme le couvercle d'appareil.

13. Système de test selon l'une des revendications 9 à 12, **caractérisé en ce qu'**un ressort de pression (66) de l'agencement à ressorts (26) s'applique sur une bobine réceptrice (54) de la cassette à bande (12).

14. Système de test selon l'une des revendications 9 à 13, **caractérisé en ce que** l'agencement à ressorts (26) comprend au moins un ressort de pression (46) susceptible de s'écarter transversalement à l'axe d'insertion, pour la réalisation d'une liaison par serrage de la cassette à bande (12).

15. Système de test selon l'une des revendications 9 à 14, **caractérisé en ce que** la cassette à bande (12) mise en place est sollicitée élastiquement par l'agencement à ressorts (26) à au moins deux points d'appui espacés.

16. Système de test selon l'une des revendications 1 à 15, **caractérisé en ce que** la cassette à bande (12) peut être reliée à un entraînement de bande par l'intermédiaire d'un accouplement, et **en ce que** ledit accouplement comprend des éléments à correspondance de forme (32, 64), susceptibles d'être mis en prise l'un avec l'autre, de manière solidaire en rotation, par un mouvement d'évitement lors de la mise en place de la cassette à bande (12).

17. Cassette à bande (12) comprenant une bobine débitrice (52) et une bobine réceptrice (54) ainsi qu'un guide-bande (56) servant au transport défini d'une bande de test analytique (50) entre lesdites bobines (52, 54), conçue en tant que consommable pour un système de test selon l'une des revendications précédentes.
